(19) Europäisches Patentamt
European Patent Office
Office européen des brevets

(11) **EP 1 200 106 B2**

(12) **NEW EUROPEAN PATENT SPECIFICATION**
After opposition procedure

(45) Date of publication and mention
of the opposition decision:
**11.08.2010 Bulletin 2010/32**

(45) Mention of the grant of the patent:
**07.05.2003 Bulletin 2003/19**

(21) Application number: **00943697.3**

(22) Date of filing: **10.07.2000**

(51) Int Cl.:
*A61K 36/185* (2006.01)     *A61P 29/00* (2006.01)
*A61P 37/00* (2006.01)     *A61P 17/06* (2006.01)
*A61P 17/00* (2006.01)     *A61P 19/02* (2006.01)

(86) International application number:
**PCT/DK2000/000383**

(87) International publication number:
**WO 2001/003712 (18.01.2001 Gazette 2001/03)**

(54) **COMPOSITION CONTAINING EXTRACTS OF BUTYROSPERMUM PARKII AND THE USE AS MEDICAMENT**

ZUSAMMENSETZUNG ENTHALTEND BUTYROSPERMUM PARKII EXTRAKTE UND DIE VERWENDUNG ALS ARZNEIMITTEL

COMPOSITION CONTENANT DES EXTRAITS DE BUTYROSPERMUM PARKII ET UTILISATION EN TANT QUE MEDICAMENT

(84) Designated Contracting States:
**AT BE CH CY DE DK ES FI FR GB GR IE IT LI LU MC NL PT SE**

(30) Priority: **09.07.1999 DK 100399**
**16.09.1999 DK 132399**
**20.09.1999 US 154651 P**
**16.03.2000 DK 200000434**
**21.03.2000 US 190919 P**

(43) Date of publication of application:
**02.05.2002 Bulletin 2002/18**

(60) Divisional application:
**03076393.2 / 1 354 595**

(73) Proprietor: **BSP Pharma A/S**
**2100 Copenhagen O (DK)**

(72) Inventor: **WEIDNER, Morten, Sloth**
**DK-2830 Virum (DK)**

(74) Representative: **Plougmann & Vingtoft A/S**
**Sundkrogsgade 9**
**P.O. Box 831**
**2100 Copenhagen Ø (DK)**

(56) References cited:
**WO-A1-96//03137     WO-A1-99//63031**
**WO-A2-98//01126     FR-A- 2 770 400**
**GB-A- 932 662     US- - 5 679 393**

• **KWEIFIO-OKAI G. ET AL.: 'Antiarthritic mechanisms of amyrin triterpenes' RESEARCH COMMUNICATIONS IN MOLECULAR PATHOLOGY AND PHARMACOLOGY vol. 85, no. 1, July 1994, pages 45 - 55**
• **Report of Analysis No. 04-059, Lipex Shea-U, 06.02.2004, Karlshamns AB**
• **Karlshamns products: Lipids in cosmetics, Karlshamns AB, April 1999**

**Description**

**FIELD OF THE INVENTION**

The present invention relates to the use of a pharmaceutical composition for oral administration comprising an extract or a concentrate of *Butyrospermum parkii* for the manufacture of a medicament for the treatment or prevention of a chronic inflammatory disease in a mammal.

**BACKGROUND OF THE INVENTION**

Hypersensitivity is defined as a state of altered reactivity in which the body reacts with an exaggerated immune response to a substance (antigen). Hypersensitivity may be caused by exogenous or endogenous antigens.

Hypersensitivity reactions underlie a large number of diseases. Among these, allergic and autoimmune conditions are of great importance. A classification of hypersensitivity diseases is given in the textbook Clinical Medicine (Kumar, P. and Clark, M.: "Clinical Medicine", 3rd edition, p. 147-150, 1994, Bailliere Tindall, London).

Type I hypersensitivity reactions (IgE mediated allergic reactions) are caused by allergens (specific exogenous antigens), e.g. pollen, house dust, animal dandruff, moulds, etc. Allergic diseases in which type I reactions play a significant role include asthma, eczema (atopic dermatitis), urticaria, allergic rhinitis and anaphylaxis.

Type II hypersensitivity reactions are caused by cell surface or tissue bound antibodies (IgG and IgM) and play a significant role in the pathogenesis of myasthenia gravis, Good-pasture's syndrome and Addisonian pernicious anaemia.

Type III hypersensitivity reactions (immune complex) are caused by autoantigens or exogenous antigens, such as certain bacteria, fungi and parasites. Diseases in which type III hypersensitivity reactions play a significant role include lupus erythematosus, rheumatoid arthritis and glomerulonephritis.

Type IV hypersensitivity reactions (delayed) are caused by cell or tissue bound antigens. This type of hypersensitivity plays a significant role in a number of conditions, e.g. graft-versus-host disease, leprosy, contact dermatitis and reactions due to insect bites.

A number of drug classes are available for the treatment of hypersensitivity reactions. Among these the corticosteroids are some of the most widely used drugs. Corticosteroids primarily exert their pharmacological action by non-selectively inhibiting the function and proliferation of different classes of immune cells resulting in suppression of hypersensitivity reactions. Unfortunately, the corticosteroids are associated with a number of serious side effects, e.g. immuno-suppression, osteoporosis and skin atrophy.

The African tree *Butyrospermum pakii*, commonly known as the Karite tree, grows wild in the dry parts of equatorial central Africa. The fruits of this tree contain a nut which is commonly known as the shea nut. The nuts are 3-4 cm long and have an oil content of approximately 50%. The major components of the oil are triglycerides, but the oil also contains several percent of an unsaponifiable fraction consisting of polyisoprenic hydrocarbons (karitene), triterpene alcohols and sterols. The oil is commonly known as shea butter. The characteristic triterpene alcohols of Butyrospermum (in the present invention termed Butyrospermum-triterpenes) are lupeol, parkeol, germanicol, dammaradienol, 24-methylene-dammarenol, butyrospermol, $\alpha$-amyrin and $\beta$-amyrin, and esters thereof, especially cinnamic acid, acetic acid or fatty acid esters.

Ordinary shea butter contains 1-5% (w/w) Butyrospermum-triterpenes and with a dosage of shea butter of 1 -20% (w/w) in existing topical cosmetic or pharmaceutical products, the contents of Butyrospermum-triterpenes in such products would be maximum 1% (w/w).

FR 2770400 (WO 99/22706) discloses cosmetic or dermopharmaceutical compositions containing an extract of the flowers of *Butyrospermum parkii.* The patent does not specify the chemical components of the flowers, but to the inventors best knowledge the flowers do not contain any substantial amounts of Butyrospermum-triterpenes.

Shea butter is widely used as an emollient in cosmetic products and to a minor extent as the fatty phase of topical pharmaceutical products, such as ointments and creams.

GB 932662 discloses pharmaceutical compositions comprising butyrospermol. According to the patent butyrospermol may be obtained from *Butyrospermum parkii.*

The present invention relates to the use of a pharmaceutical composition comprising an extract or concentrate of Butyrospermum parkii comprising at least 5% (w/w) of a Butyrospermum-triterpene fraction such that said composition comprises at least 5% (w/w) of said Butyrospermum-triterpene fraction,

said Butyrospermum-triterpene fraction comprises:

- at least 2% (w/w) lupeol;
- at least 2% (w/w) $\alpha$-amyrin and/or $\beta$-amyrin;
- at least 2% (w/w) butyrospermol; and
- optionally at least 1% germanicol, dammaradienol, 24-methylene-dammarenol d/or parkeol, wherein said triterpenes are In the form of free alcohols or esters thereof, for the manufacture of a medicament for the treatment or prevention of a chronic inflammatory disease In a mammal, and wherein said composition is administered orally.

**DETAILED DESCRIPTION OF THE INVENTION**

It has been found by the present inventor that a pharmaceutical composition as described herein significantly suppresses inflammation when administered orally.

In example 1 the anti-inflammatory effect of a composition according to the invention was measured in a well-established model of hypersensitivity (arthritis) in the mouse. In this experiment the composition exerted a significant effect (at 50 mg/kg) comparable to that of cyclophosphamide (at 10 mg/kg). In a separate experiment (see example 4) the $LD_{50}$ in the rat of the same composition was shown to be above 2000 mg/kg, while the $LD_{50}$ in the rat of cyclophosphamide is 94 mg/kg (The Merck Index, 13th edition, 1989, Merck and Co Inc.). Thus, the therapeutic index of the composition is far superior to that of cyclophosphamide.

The compositions for oral administration provide a surprisingly good anti-inflammatory effect with a surprisingly good safety profile. Thus, the compositions are virtually non-toxic and yet very therapeutically effective. The present inventor puts forward the hypothesis that the very beneficial therapeutic index of the compositions compared to single chemical anti-hypersensitivity drugs is due to the more complex nature of the compositions, giving a lower toxic load on the body of any single chemical compound and yet giving a surprisingly good therapeutic effect, due to synergistic effects between the components of the compositions.

The pharmaceutical composition to be used according to the invention has a weight percentage (w/w) of the Butyrospermum-triterpene fraction in the composition of at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%; e.g. at least 85%, such as at least 90%, e.g. at least 95%, or at least 96%, such as at least 97%, e.g. at least 98%, such as at least 99%, e.g. at least 100%; furthermore the weight percentage (w/w) of the Butyrospermum-triterpene fraction in the composition typically comprises at most 100%, e.g. 99%, at most 98%, e.g. at most 97%, e.g. at most 96, e.g. at most 95%, e.g. at most 90%, e.g. at most 85%, e.g. at most 80%, e.g. at most 75%, e.g. at most 70%, at most 65%, e.g. at most 60%, e.g. at most 55%, e.g. at most 50%, e.g. at most 45%, at most 40%, e.g. at most 35%, e.g. at most 30%, e.g. at most 25%, e.g. at most 20%, e.g. at most 15%, e.g. at most 10%, e.g. at most 9%, e.g. at most 8%, e.g. at most 7%, e.g. at most 6%, e.g. at most 5%; the weight percentage (w/w) of the Butyrospermum-triterpene fraction in the composition typically may be in the range of 5-100%, such as in the range of 6-98%, such as in the range of 7-96%, e.g. in the range of 8-94%, such as in the range of 9-92%, such as in the range of 10-90%, e.g. in the range of 12-88%, e.g. in the range of 14-86%, such as in the range of 16-84%, such as in the range of 18-82%, e.g. in the range of 20-80%

wherein the Butyrospermum-triterpene fraction is comprised of

- at least 2% (w/w) lupeol, e.g. at least 3%, such as at least 4%, e.g. at least 5%, e.g. at feast 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%; or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;

- at least 2% (w/w) α-amyrin and/or β-amyrin, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;

- at least 2% (w/w) butyrospermol, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%;

- and optionally at least 1% (w/w) germanicol, dammaradienol, 24-methylene-dammarenol and/or parked, e.g. at least 2%, e.g. at least 3%, such as at least 4%, e.g. at least 5%, e.g. at least 6%, at least 7%, at least 8%, such as at least 9%, e.g. at least 10%, such as at least 15%, e.g. at least 20%, such as at least 25%, or at least 30%, e.g. at least 35%, e.g. at least 40%, at least 45%, or at least 50%, such as at least 55%, e.g. at least 60%, such as at least 65%, e.g. at least 70%, at least 75%, such as at least 80%, e.g. at least 85%, such as at least 90%, e.g. at least 95%, or at least 96%, such as at least 97%, e.g. at least 98%, such as at least 99%, e.g. at least 100%.

In a preferred embodiment, the Butyrospermum-triterpene fraction comprises:

- 10-40% (w/w) lupeol;

- 10-40% (w/w) α-amyrin and/or β-amyrin;
- 10-40% (w/w) butyrospermol; and
- optionally 1-30% germanicol, dammaradienol, 24-methylene-dammarenol and/or parkeol,

wherein said triterpenes may be in the form of free alcohols or esters thereof, especially cinnamic acid, acetic acid or fatty acid esters; and the Butyrospermum-triterpene fraction

Accordingly, the pharmaceutical composition to be used according to the invention may contain or a weight percentage (w/w) of the Butyrospermum-triterpene lupeol in the composition in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range, of 6-45%, such as in the range of 7-40%, such as in the range of 8-40%, such as in the range of 9-40%, such as in the range of 10-40%; the weight percentage (w/w) of the Butyrospermum-triterpene α-amyrin and/or β-amyrin in the composition typically may be in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range of 6-45%, such as in the range of 7-40%, such as in the range of 8-40%, such as in the range of 9-40%, such as in the range of 10-40%; the weight percentage (w/w) of the Butyrospermum-triterpene butyrospermol in the composition typically may be in the range of 2-95%, such as in the range of 3-95%, such as in the range of 4-90%, e.g. in the range of 5-90%, such as in the range of 5-80%, such as in the range of 5-75%, e.g. in the range of 5-70%, e.g. in the range of 5-65%, such as in the range of 5-60%, such as in the range of 5-55%, e.g. in the range of 5-50%, such as in the range of 6-45%, such as in the range of 7-40%, such as in the range of 8-40%, such as in the range of 9-40%, such as in the range of 10-40%; and optionally the weight percentage (w/w) of the Butyrospermum-triterpene(s) germanicol, dammaradienol, 24-methylene-dammarenol and/or parked in the composition typically may be in the range of 1-95%, such as in the range of 1-90%, such as in the range of 1-80%, e.g. in the range of 1-70%, such as in the range of 1-60%, such as in the range of 2-50%, e.g. in the range of 2-40%, e.g. in the range of 2-35%, such as in the range of 2-30%.

In the present invention the "Butyrospermum-triterpene fraction" is defined as a fraction comprising at least one triterpene selected from the group consisting of lupeol, parkeol, germanicol, dammaradienol, 24-methylene-dammarenol, butyrospermol, α-amyrin and β-amyrin, and esters thereof, especially cinnamic acid or acetic acid esters.

The "sterol fraction" is defined as a fraction comprising at least one sterol selected from the group consisting of stigmasterol, avanasterol, 24-methyl-cholest-7-enol, karitesterol A, karitesterol B and α-spinasterol. The composition may contain one or more of these, such as 2, 3, 4, 5, 6, 7 or all of the sterols listed above wherein said sterols may be in the form of free alcohols or esters thereof, especially cinnamic acid, acetic acid or fatty acid esters.

The extractor concentrate of *Butyrospermum parkii* may be derived from any part of the plant, such as the fruit (nut), leaves, stem, bark or root. Preferably the extract or concentrate is derived from the fruit. Furthermore, the extract or concentrate may be derived from the oil or fat (shea butter) derived from the fruit of *Butyrospermum parkii* by any method of extraction or fractionation, e.g. comprising the unsaponifiable fraction. Preferably, the triterpene alcohols and the sterols are derived in from the unsaponifiable fraction of the oil or fat from *Butyrospermum* parkii.

Extracts or concentrates according to the invention can i.a. be obtained by extraction or distillation (e.g. hydro, steam or vacuum distillation) of fresh or dried *Butyrospermum parkii* or parts thereof, preferably the nut. Extraction may be performed with a number of different organic solvents. The extraction can be performed hot or cold by the employment of any extraction technology e.g. maceration, percolation or supercritical extraction (e.g. with carbon dioxide).

The preferred extraction solvents are acetone, methyl ethyl ketone, methyl acetate, ethyl acetate, lower alkanols having 1-4 carbon atoms, pentane, hexane, heptane and mixtures thereof. The preferred extraction temperature is close to the boiling point of the employed solvent due to extraction efficacy, but lower temperatures are also applicable, a longer period of extraction then being necessary.

By changing the composition of the applied solvent, the extraction can be made more selective for certain constituents of *Butyrospermum parkii* thus enhancing or reducing the contents thereof in the finished extract or concentrate.

After the primary extraction process, a second step of processing, such as liquid-liquid extraction, column chromatography or any type of distillation, can be employed to remove or to concentrate any constituent of the extract. Hereby any constituent of *Butyrospermum* parkii can be avoided or concentrated in the finished extract. Thus the content of any component can be standardised to obtain a composition and the ratio between the different Butyrospermum-triterpenes may be varied dramatically in the pharmaceutical compositions described herein and in specific cases any of the Butyrospermum-triterpenes and any number of the Butyrospermum-triterpenes may be excluded from a specific composition.

The above mentioned pharmacological actions provide part of the rationale for the following therapeutic applications of a composition comprising an extract or a concentrate of *Butyrospermum parkii* as described above and, optionally, a pharmaceutically acceptable carrier for oral administration:

The treatment or prevention of chronic inflammation characterised by oral administration of the above mentioned compositions. The therapeutic action may be relevant to all known diseases associated chronic inflammation. The following examples are not limiting with respect to this: psoriasis, contact dermatitis, atopic dermatitis, Crohn's Disease, Ulcerative Colitis, Rheumatoid Arthritis, and osteoarthritis.

According to the invention the above mentioned compositions can be combined with any other active ingredient(s) to potentiate the therapeutic action.

A pharmaceutical acceptable carrier for oral administration can be water or vehicles other than water, said other vehicles can be used in the compositions and can include solids or liquids such as solvents, thickeners and powders. Examples of each of these types of vehicles, which can be used singly or as compositions of one or more vehicles, are as follows:

Emollients, such as stearyl alcohol, glyceryl monoricinoleate, glyceryl monostearate, propane-1,2-diol, butane-1,3-diol, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polyethylene glycol, triethylene glycol, lanolin, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

solvents, such as water, methylene chloride, isopropanol, castor oil, ethylene glycol monoethyl ether, diethylene glycol monobutyl ether, diethylene glycol monoethyl ether, dimethyl sulfoxide, tetrahydrofuran, vegetable and animal oils, glycerol, ethanol, propanol, propylene glycol, and other glycols or alcohols, fixed oils;

humectants or moistening agents, such as glycerin, sorbitol, sodium 2-pyrrolidone-5-carboxylate, soluble collagen, dibutyl phthalate, gelatin;

powders, such as chalk, talc, kaolin, starch and derivatives thereof, gums, colloidal silicon dioxide, sodium polyacrylate, chemically modified magnesium aluminium silicate, hydrated aluminium silicate, carboxyvinyl polymer, sodium carboxymethyl cellulose, ethylene glycol monostearate;

gelling or swelling agents, such as pectin, gelatin and derivatives thereof, cellulose derivatives such as methyl cellulose, carboxymethyl cellulose or oxidised cellulose, cellulose gum, guar gum, acacia gum, karaya gum, tragacanth gum, beritonite, agar, alginates, carbomer, gelatine,. bladderwrack, ceratonia, dextran and derivatives thereof, ghatti gum, hectorite, ispaghula husk, xanthan gurn;

polymers, such as polylactic acid or polyglycolic acid polymers or copolymers thereof, paraffin, polyethylene, polyethylene oxide, polyethylene glycol, polypropylene glycol, polyvinylpyrrolidone;

surfactants, such as non-ionic surfactants, e.g. glycol and glycerol esters, macrogol ethers and esters, sugar ethers and esters, such as sorbitan esters, ionic surfactants, such as amine soaps, metallic soaps, sulfated fatty alcohols, alkyl ether sulfates, sulfated oils, and ampholytic surfactants and lecitins;

buffering agents, such as sodium, potassium, aluminium, magnesium or calcium salts (such as the chloride, carbonate, bicarbonate, citrate, gluconate, lactate, acetate, gluceptate or tartrate).

Furthermore, it is obvious that in the use according to the invention for the preparation of medicaments, the above mentioned compositions may be mixed with additives such as surfactants, solvents, thickeners, stabilisers, preservatives, antioxidants, flavours, etc. to obtain a desirable product formulation suitable for oral administration. The route of administrations is oral and the following examples are not limiting with respect to this: tablets, capsules, lozenges, fluids, granulates, emulsions, etc. Optionally, the composition may also contain surfactants such as bile salts, polyoxyethylene-sorbitan-fatty acid esters or polyalcohol mixed chain-length fatty acid esters for improving dispersibility of the composition in the digestive fluids leading to improved bioavailability or for obtaining the final dosage form of the composition.

## EXAMPLES

### Example 1

Summary of the study

BPC, a concentrate of Butyrospermum parkii as described herein, was evaluated for possible anti-inflammatory activity in BALB/c mouse arthritis induced by collagen monoclonal antibody (mAb) and lipopolysaccharide. The test substance was administered orally once daily for 3 consecutive days. Significant reduction relative to the vehicle treated control group of hind paw edema was observed at 50 mg/kg x 3 (57%, 58%, 67% and 78%) at day 7, 10, 14 and 17. Concurrently tested cyclophosphamide at 10 mg/kg x 3 provided a 79%, 91% and 90% reduction of hind paw edema relative to the vehicle-treated control group at day 10, 14 and 17.

Test substance

A composition as described herein was prepared by fractionation of shea butter and subsequently diluting the obtained concentrate of *Butyrospermum parkii* in corn oil. The applied concentrate of *Butyrospermum* (termed BPC in the following) contained 26% of a Butyrospermum-triterpene fraction (primarily in the form of cinnamic acid esters) selected from the group consisting of butyrospermol, lupeol, parkeol, germanicol, dammaradienol, 24-methylene-dammarenol, α-amyrin and β-amyrin, comprising the following specific Butyrospermum-triterpene amounts:

- 23% lupeol;
- 39% α-amyrin and β-amyrin; and
- 26% butyrospermol

Furthermore the concentrate contained 2.2% sterols selected from the group consisting of stigmasterol, avanasterol, 24-methyl-cholest-7-enol, karitesterol A, karitesterol B and α-spinasterol.

Dosing pattern

BPC was dissolved in 100% corn oil. The test substance was administered orally once daily for 3 consecutive days at 50 mg/kg, as well as positive control of 10 mg/kg for cyclophosphamide. Dosing volume was 5 ml/kg.

Animals

In this study, male BALB/c mice weighing 20 ± 1 grams were used. Space allocation for 5 mice was 45 x 23 x 15 cm. The animals were housed in APEC® (Allentown Gaging, Allentown, NJ 08501, U. S. A.) cages and maintained in a hygienic environment under controlled temperature (22°C - 24°C) and humidity (60% - 80%) with 12-hours light/dark cycles for at least one week prior to being used. Free access to standard lab chow for mice and tap water was granted. All aspects of this work including housing, experimentation and disposal of animals were performed in general according to the International Guiding Principles for Biomedical Research Involving Animals (CIOMS Publication No. ISBN 9290360194, 1985).

Chemicals

The chemicals employed in the present study were Corn Oil (Sigma, U. S. A.), Cyclophosphamide (Sigma, U. S. A.), Lipopolysaccharide (Sigma, U. S. A.), and Arthrogen-CIA™ Monoclonal Antibodies D8, F10, DI-2G and A2 (Chondrex, U. S. A.).

Equipment

Equipment employed was a Plethysmometer (Ugo Basile, U. S. A.),

Method

The study was performed according to the method of Terato et al (. Autoimmunity, 22: 137-147, 1995).
Groups of 5 BALB/c mice, 6-8 weeks of age, were used for the induction of arthritis by monoclonal antibodies (mAbs) and lipopolysaccharide (LPS). The animals were administered intravenously of a combination of 4 different mAbs (D8, F10, DI-2G and A2) in a total of 4 mg/mouse at day O. This was followed by intravenous 25 μg of LPS 72 hours later (day 3). From day 3, test substances were administered orally once daily for 3 consecutive days. At day 5, one or two paws (particularly the hind) began to appear red and swollen, and from day 7, arthritis symptoms of the two hind paws became severely red and swollen in untreated mice. A plethysmometer (Ugo Basile Cat # 7150) with water cell (12 mm diameter) was used for the measurement of increase in both hind paw volumes at day 7, 10, 14 and 17. The percent of inhibition of increased paw volume was calculated as follows:

$$\text{Inhibition (\%): } [1 - (Tn - To)/(Cn - Co)] \times 100$$

Where:

Co (Cn): Volume of day 0 (day n) in vehicle control (both hind paws summed)
To (Tn): Volume of day 0 (day n) in test compound-treated group (both hind paws summed)

Statistics

Wilcoxon rank sum test for paired differences was used for comparing swelling in the test compound treated groups with swelling in the control group.

Results

Reduction of swelling relative to the vehicle treated control group of hind paw edema was observed at 50 mg/kg x 3 (57%, 58%, 67% and 78%) at day 7, 10, 14 and 17. The inhibition was statistically significant (p<0.05, Wilcoxon) at day 10, 14 and 17. Concurrently tested cyclophosphamide at 10 mg/kg x 3 provided a statistically significant (p<0.05, Wilcoxon) 79%, 91% and 90% reduction of hind paw edema relative to the vehicle-treated control group at day 10, 14 and 17.

## Example 2

### Summary

BPC, a concentrate of Butyrospermum parkii as described herein, was evaluated for acute oral toxicity in the rat. At a dose of 2000 mg/kg, BPC was found not to produce toxicity or mortality. Thus it was concluded that the $LD_{50}$ was above 2000 mg/kg body weight.

### Test substance

A composition as described herein was prepared by fractionation of shea butter and subsequently diluting the obtained concentrate of *Butyrospermum parkii* in corn oil. The applied concentrate of *Butyrospermum* (termed BPC in the following) contained 33% of a Butyrospermum-triterpene fraction comprising:

- 26% (w/w) lupeol;
- 44% (w/w) α-amyrin and/or β-amyrin; and
- 30% (w/w) butyrospermol;

Furthermore the concentrate contained 2.7% of a sterol fraction comprising:

- 43% α-spinasterol;
- 37% stigmasterol; and
- 11% avanasterol.

### Study description

The acute oral toxicity in rats was determined according to the method recommended in the OECD guideline No 420, "Acute Oral Toxicity - Fixed Dose Method", July 1992 and the EEC Directive published in: "Official Journal of the European Communities" No: L 383A. volume 35, 29.12.1 992, part B1 "Acute Toxicity (Oral) - Fixed Dose Method".

The study was initiated with a sighting study, in which one female rat was given 2000 mg BPC/kg body weight. No clinical signs of toxicity were observed in this rat.

On the basis of the results of the sighting study the main study was carried out with one group consisting of 5 female rats given a dose of 2000 mg BPC/kg body weight.

All animals in the main study survived the treatment and showed no signs of evident toxicity.

The rats had a normal body weight gain during the study period.

Under the experimental conditions described in this report, it was found that the dose level tested (2000 mg BPC/kg body weight), highest required dose level, did not produce mortality. The minimal lethal dose was above 2000 mg BPC/kg body weight.

## Example 3

### Summary

A randomised, double-blind and placebo controlled phase II clinical study is performed in patients suffering from rheumatoid arthritis to test the safety and efficacy of a concentrate of Butyrospermum parkii as described herein.

### Test substance

A composition as described herein is prepared by fractionation of shea butter and subsequently formulating the obtained concentrate of *Butyrospermum parkii* in soft gelatine capsules each containing 750 mg of the concentrate. The applied concentrate of *Butyrospermum* (termed BPC in the following) contains 33% of a Butyrospermum-triterpene fraction comprising:

- 26% (w/w) lupeol;
- 44% (w/w) α-amyrin and/or β-amyrin; and
- 30% (w/w) butyrospermol;

Furthermore the concentrate contained 2.7% of a sterol fraction comprising:

- 43% α-spinasterol;
- 37% stigmasterol; and
- 11% avanasterol.

A placebo capsule is prepared with exactly the same appearance and weight.

### Study purpose

To test the efficacy and safety of 1500 mg of BPC daily for 18 weeks, compared to placebo, in patients with rheumatoid arthritis.

### Study design

Randomised, double blind, placebo controlled in two parallel groups.

### Study population

40 patients, both genders, with diagnosed rheumatoid arthritis. The patients are allowed to continue with their existing medication.

### Study plan

At visit 1 patients fulfilling the inclusion criteria and passing the exclusion criteria are randomised into the following groups:

- BPC 750 mg x 2 daily

- Placebo x 2 daily

At visit 1, 2 (6 weeks), 3 (12 weeks) and 4 (18 weeks) the status of the patient is evaluated using the recognised Stanford Health Assessment Questionnaire (HAQ).

## Claims

1. Use of a pharmaceutical composition comprising an extract or concentrate of Butyrospermum parkii comprising at least 5% (w/w) of a Butyrospermum-triterpene fraction such that said composition comprises at least 5% (w/w) of said Butyrospermum-triterpene fraction,
said Butyrospermum-triterpene fraction comprises:

   - at least 2% (w/w) lupeol;
   - at least 2% (w/w) $\alpha$-amyrin and/or $\beta$-amyrin;
   - at least 2% (w/w) butyrospermol; and
   - optionally at least 1% germanicol, dammaradienol, 24-methylene-dammarenol and/or parkeol, wherein said triterpenes are in the form of free alcohols or esters thereof, for the manufacture of a medicament for the treatment or prevention of a chronic inflammatory disease in a mammal, and wherein said composition is administered orally.

2. The use according to claim 1, wherein said Butyrospermum-triterpene fraction comprises:

   - 10-40% (w/w) lupeol;
   - 10-40% (w/w) $\alpha$-amyrin and/or $\beta$-amyrin;
   - 10-40% (w/w) butyrospermol; and
   - optionally 2-30% germanicol, dammaradienol, 24-methylene-dammarenol and/or parkeol, wherein said triterpenes are in the form of free alcohols or esters thereof.

3. The use according to any one of claims 1 or 2, wherein the extract or concentrate of Butyrospermum parkii further comprises a sterol fraction comprising at least one sterol selected from the group consisting of stigmasterol, avanasterol, 24-methyl-cholest-7-enol, karitesterol A, karitesterol B and $\alpha$-spinasterol.

4. The use according to claim 3, wherein said sterols are in the form of free alcohols or esters thereof.

5. The use according to claim 1, wherein said esters are selected from the group consisting of cinnamic acid esters, acetic acid esters and fatty acid esters.

6. The use according to claim 4, wherein said esters of sterols are selected from the group consisting of cinnamic acid esters, acetic acid esters and fatty acid esters.

7. The use according to any one of the preceding claims, wherein the Butyrospermum-triterpene fraction and the sterol fraction comprises up to 100% (w/w) of the extract or concentrate of Butyrospermum parkii.

8. The use according to any one of the preceding claims, further comprising a pharmaceutically acceptable carrier.

9. The use according to claim 8, wherein the pharmaceutical composition is formulated in a capsule.

10. The use according to any of the preceding claims, wherein said chronic inflammatory disease is selected from the group consisting of psoriasis, atopic dermatitis, contact dermatitis, Crohn's disease, ulcerative colitis, rheumatoid arthritis and osteoarthritis.

11. The use according to claim 10, wherein said chronic inflammatory disease is rheumatoid arthritis or osteoarthritis.

12. The use according to claim 11, wherein said chronic inflammatory disease is osteoarthritis.

## Patentansprüche

1. Verwendung einer pharmazeutischen Zusammensetzung, umfassend ein Extrakt oder ein Konzentrat von Butyrospermum parkii, umfassend mindestens 5 Gew.-% einer Butyrospermum-Triterpen-Fraktion, sodass die Zusammensetzung mindestens 5 Gew.-% der Butyrospermum-Triterpen-Fraktion umfasst, wobei die Butyrospermum-Triterpen-Fraktion umfasst:

   - mindestens 2 Gew.-% Lupeol;
   - mindestens 2 Gew.-% $\alpha$-Amyrin und/oder $\beta$-Amyrin;
   - mindestens 2 Gew.-% Butyrospermol; und
   - gegebenenfalls mindestens 1 % Germanicol, Dammaradienol, 24-Methylendammarenol und/oder Parkeol, wobei die Triterpene in Form von freien Alkoholen oder Estern davon vorliegen, zur Herstellung eines Arzneimittels zur Behandlung oder Prävention einer chronischen entzündlichen Krankheit bei einem Säugetier und wobei die Zusammensetzung oral verabreicht wird.

2. Verwendung nach Anspruch 1, wobei die Butyrospermum-Triterpen-Fraktion umfasst:

   - 10-40 Gew.-% Lupeol;

- 10-40 Gew.-% $\alpha$-Amyrin und/oder $\beta$-Amyrin;
- 10-40 Gew.-% Butyrospermol; und
- gegebenenfalls 2-30 % Germanicol, Dammaradienol, 24-Methylendammarenol und/oder Parkeol, wobei die Triterpene in Form von freien Alkoholen oder Estern davon vorliegen.

3. Verwendung nach einem der Ansprüche 1 oder 2, wobei das Extrakt oder Konzentrat von Butyrospermum parkii weiterhin eine Sterolfraktion umfasst, umfassend mindestens ein Sterol ausgewählt aus der Gruppe, bestehend aus Stigmasterol, Avanasterol, 24-Methylcholest-7-enol, Karitesterol A, Karitesterol B und $\alpha$-Spinasterol.

4. Verwendung nach Anspruch 3, wobei die Sterole in Form von freien Alkoholen oder Estern davon vorliegen.

5. Verwendung nach Anspruch 1, wobei die Ester ausgewählt sind aus der Gruppe, bestehend aus Zimtsäureestern, Essigsäureestern und Fettsäureestern.

6. Verwendung nach Anspruch 4, wobei die Ester der Sterole ausgewählt sind aus der Gruppe, bestehend aus Zimtsäureestern, Essigsäureestern und Fettsäureestern.

7. Verwendung nach einem der vorhergehenden Ansprüche, wobei die Butyrospermum-Triterpen-Fraktion und die Sterolfraktion bis zu 100 Gew.-% des Extrakts oder des Konzentrats von Butyrospermum parkii umfasst.

8. Verwendung nach einem der vorhergehenden Ansprüche, weiterhin umfassend einen pharmazeutisch verträglichen Träger.

9. Verwendung nach Anspruch 8, wobei die pharmazeutische Zusammensetzung als eine Kapsel formuliert ist.

10. Verwendung nach einem der vorhergehenden Ansprüche, wobei die chronische entzündliche Krankheit ausgewählt ist aus der Gruppe, bestehend aus Psoriasis, atopischer Dermatitis, Kontaktdermatitis, Morbus Crohn, Colitis ulcerosa, rheumatoide Arthritis und Osteoarthritis.

11. Verwendung nach Anspruch 10, wobei die chronische entzündliche Krankheit rheumatoide Arthritis oder Osteoarthritis ist.

12. Verwendung nach Anspruch 11, wobei die chronische entzündliche Krankheit Osteoarthritis ist.

## Revendications

1. Utilisation d'une composition pharmaceutique comprenant un extrait ou un concentré de Butyrospermum parkii comprenant au moins 5 % (p/p) d'une fraction à base de triterpènes de Butyrospermum de telle façon que ladite composition comprenne au moins 5 % (p/p) de ladite fraction à base de triterpènes de Butyrospermum,

   ladite fraction à base de triterpènes de Butyrospermum comprenant :

   - au moins 2 % (p/p) de lupéol ;
   - au moins 2 % (p/p) d'$\alpha$-amyrine et/ou de $\beta$-amyrine ;
   - au moins 2 % (p/p) de butyrospermol ; et
   - éventuellement au moins 1 % de germanicol, de dammaradiénol, de 24-méthylène-dammarénol et/ou de parkéol, dans laquelle lesdits triterpènes sont sous la forme d'alcools libres ou d'esters de ceux-ci, pour la fabrication d'un médicament destiné au traitement ou à la prévention d'une maladie inflammatoire chronique chez un mammifère, et dans laquelle ladite composition est administrée par voie orale.

2. L'utilisation selon la revendication 1, dans laquelle ladite fraction à base de triterpènes de Butyrospermum comprend :

   - 10 à 40 % (p/p) de lupéol ;
   - 10 à 40 % (p/p) d'$\alpha$-amyrine et/ou de $\beta$-amyrine ;
   - 10 à 40 % (p/p) de butyrospermol ; et
   - éventuellement 2 à 30 % de germanicol, de dammaradiénol, de 24-méthylène-dammarénol et/ou de parkéol, dans laquelle lesdits triterpènes sont sous la forme d'alcools libres ou d'esters de ceux-ci.

3. L'utilisation selon l'une quelconque des revendications 1 ou 2, dans laquelle l'extrait ou le concentré de Butyrospermum parkii comprend également une fraction à base de stérol comprenant au moins un stérol choisi dans le groupe consistant en stigmastérol, avanastérol, 24-méthyl-cholest-7-énol, karitestérol A, karitestérol B et $\alpha$-spinastérol.

4. L'utilisation selon la revendication 3, dans laquelle lesdits stérols sont sous la forme d'alcools libres ou d'esters de ceux-ci.

5. L'utilisation selon la revendication 1, dans laquelle lesdits esters sont choisis dans le groupe consistant en esters d'acide cinnamique, esters d'acide acétique et esters d'acide gras.

6. L'utilisation selon la revendication 4, dans laquelle lesdits esters de stérols sont choisis dans le groupe consistant en esters d'acide cinnamique, esters d'acide acétique et esters d'acide gras.

7. L'utilisation selon l'une quelconque des revendications précédentes, dans laquelle la fraction à base de triterpènes de Butyrospermum et la fraction à base stérol comprennent jusqu'à 100 % (p/p) de l'extrait ou du concentré de Butyrospermum parkii.

8. L'utilisation selon l'une quelconque des revendications précédentes, comprenant également un véhicule pharmaceutiquement acceptable.

9. L'utilisation selon la revendication 8, dans laquelle la composition pharmaceutique est formulée dans une capsule.

10. L'utilisation selon l'une quelconque des revendications précédentes, dans laquelle ladite maladie inflammatoire chronique est choisie dans le groupe consistant en psoriasis, eczéma atopique, eczéma de contact, maladie de Crohn, colite ulcéreuse, arthrite rhumatoïde et ostéoarthrite.

11. L'utilisation selon la revendication 10, dans laquelle ladite maladie inflammatoire chronique est l'arthrite rhumatoïde ou l'ostéoarthrite.

12. L'utilisation selon la revendication 11, dans laquelle ladite maladie inflammatoire chronique est l'ostéoarthrite.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2770400
- WO 9922706 A
- GB 932662 A

**Non-patent literature cited in the description**

- **Kumar, P. ; Clark, M.** *Clinical Medicine,* 1994, 147-150
- **Terato et al.** *Autoimmunity,* 1995, vol. 22, 137-147